# EUROPEAN PATENT APPLICATION

(11) **EP 3 778 880 A1**
(43) Date of publication of application: **17.02.2021**
(21) Application number: 19775992.1
(22) Date of filing: 28.03.2019
(51) Int. Cl.: C12N 7/00

(54) **METHOD FOR LARGE-SCALE PRODUCTION OF LENTIVIRUS BY USING GMP-LEVEL SERUM-FREE SUSPENSION CELLS**

(30) Priority: 29.03.2018 CN 201810273392
(71) Applicant: Cellular Biomedicine Group HK Limited, Admiralty, Hong Kong (HK)
(72) Inventor: HONG, Yi, Shanghai 201210 (CN); YAN, Ting, Shanghai 201210 (CN); YING, Jiangguo, Shanghai 201210 (CN); ZHANG, Haojie, Shanghai 201210 (CN); ZHANG, Luyi, Shanghai 201210 (CN); ZHANG, Li, Shanghai 201210 (CN)
(74) Representative: Chapman, Paul Gilmour
(86) International application number: PCT/CN2019/080215
(87) International publication number: WO 2019/184996

(57) **Abstract**

Provided is a method for large-scale production of lentivirus by using GMP-level serum-free suspension cells. Said method comprises the following steps: (a) providing a seed solution of packaging cells; (b) inoculating the seed solution in a first culture solution; (c) carrying out subculture of the packaging cells; (d) starting a liquid change operation when a liquid change trigger condition is met; (e) repeating steps (c) and (d) 1, 2 or 3 times; (f) starting a transfection operation when a transfection trigger condition is met; (g) optionally performing liquid change after transfection; (h) cultivating the transfected packaging cells; (i) starting harvesting and liquid change operations when a liquid change trigger condition is met; (j) repeating steps (h) and (i) 1, 2 or 3 times; (k) combining each of the recovered liquids; and (1) performing a purifying treatment. The culture solution used in each step is a serum-free cell culture solution.

## Description

### Technical Field

The present invention relates to the technical field of biology, particularly to a method for large-scale production of lentivirus by using GMP-level serum-free suspension cells.

### Background Technologies

Gene therapy refers to introducing exogenous therapeutic genes into target cells to correct or compensate for diseases caused by gene defects and abnormalities, or acting on disease targets through products expressed by exogenous genes to achieve the purpose of treatment.

Popular recombinant lentiviral vectors are gene therapy vectors developed based on HIV-1 (human immunodeficiency virus-1). Different from the general retroviral vectors, the recombinant lentiviral vectors have the ability to infect both dividing and non-dividing cells. The recombinant lentiviral vectors have become the preferred transgenic vectors for CART cells and gene therapy due to their high biological titer in vivo and in vitro, low immunogenicity and other advantages.

The mature HIV-1 virus is in a diameter of 100-120 nm has an icosahedral symmetrical structure and is spherical. A uniform dense cone-shaped core can be seen under an electron microscope. It contains viral RNA molecules and enzymes. The latter include reverse transcriptase, integrase and protease. The outermost layer of HIV-1 is a lipoprotein envelope. The envelope contains two glycoproteins: surface protein (gp120) and intrinsic protein (gp41). gp120 is a spike and gp41 is a transmembrane protein. The inner surface of the envelope is a matrix composed of P17, and inside the envelope is RNAO wrapped by capsid protein (P24).

The current recombinant lentiviral vectors use a genetic modification method to leave only packaging signals and target gene transcription elements in the lentiviral genome, disperse reverse transcriptase, envelope protein VSVG, gag/pol, rev, tat and other structural or regulatory genes on different vectors and meanwhile delete pathogenic genes to ensure the safety of the recombinant lentiviral vectors.

HEK293T is a cell strain derived from human embryonic renal epithelium. It is obtained from an HEK 293 cell line by transfection of adenovirus E1A gene and can express the large T antigen of SV40, and replication origin and promoter region containing SV40. The eukaryotic expression vectors of the replication origin containing SV40 virus can achieve efficient replication and transcription in HEK 293T cells, thereby increasing the expression level of exogenous genes. Therefore, HEK293T cells are widely used in lentivirus packaging and can obtain a lentivirus feed liquid with a high titer.

However, HEK293T cell as a cell strain for lentivirus production still has the following defects: 1) Large T antigen has a potential carcinogenic risk. If the downstream processes cannot remove it effectively, there will be a certain risk in clinical treatment and the large T antigen has certain immunogenicity, which will increase the difficulty of clinical treatment; 2) HEK293T is an adherent cell. It is difficult to realize industrialization through cell factory or spinner bottle production; 3) The ability to adhere to the wall is weak and if the microcarrier technology is used, cells are liable to falling off and the toxin production efficiency is significantly reduced.

Further, although some technologies have been developed to generate lentiviruses based on suspension cells, the existing production methods still have some defects. For example, they are not suitable for large-scale production and can hardly meet the strict requirements of GMP production and the virus produced has a low titer.

Therefore, there is an urgent need in this field to develop a method that can efficiently produce lentivirus on a large scale through packaging cells under serum-free and suspension culture.

### Summary

An object of the present invention is to provide a method for efficiently producing lentivirus on a large scale through packaging cells under serum-free and suspension culture.

In a first aspect of the present invention, a method for producing lentivirus from serum-free suspension cells is provided, comprising the following steps:
(a) Providing a seed solution of packaging cells, which are packaging cells that grow in suspension, for the production of lentivirus;
(b) Inoculating the seed solution to a first culture solution in a culture container to obtain a first culture, wherein the first culture solution is a serum-free cell culture solution and the inoculation density is 1 × 10⁶ to 5 × 10⁶ cells/ml and the volume of the first culture solution is 3- to 100 L (preferably 5 to 50 L);
(c) Subjecting the first culture to subculture of packaging cells under the set conditions of temperature 30°C to 38°C, dissolved oxygen 35% to 55%, CO₂ concentration 2% to 10% and pH 6.9 to 7.4;
(d) Starting a liquid change operation when a liquid change trigger condition is met,

Here, the liquid change trigger condition includes:
(s1) Real-time pH ≤6.9, preferably ≤7.0, more preferably ≤7.05;
(s2) By adjusting the concentrations of oxygen, air, nitrogen and/or CO₂, the real-time pH value still shows a downward trend; and
(s3) Subculture time ≥72 h;

Here, the liquid change operation includes: discharging the cell-free supernatant in the culture from the culture container, wherein the volume of the culture before discharge of the supernatant is Vq1, the volume of the culture after discharge of the supernatant is Vh1 and the Vql/Vhl ratio is 3 to 15 (preferably 4 to 7, more preferably 5 to 6); then replenishing the culture solution to the culture container to form a culture of packaging cells;
(e) Repeating the steps (c) and (d) n times, where n is 1, 2 or 3;
(f) Starting a transfection operation when a transfection trigger condition is met,

Here, the transfection trigger condition includes:
(t1) The total cell quantity is 0.05∼2 × 10¹¹ cells;
(t2) The cell density is 0. 5∼5 × 10⁶ cells/ml;
(t3) The cell viability is ≥90%; and
(t4) The total culture time is ≥72 h;

Here, the transfection operation includes: mixing the production plasmid and the transfection reagent for the production of the lentivirus and adding them to the culture container, thereby introducing the packaging cells and forming transfected packaging cells;
(g) Optionally performing liquid change after transfection;
(h) Cultivating the transfected packaging cells under the set conditions of temperature 30°C to 37°C, dissolved oxygen 35% to 55%, CO₂ concentration 2% to 10% and pH 6.9 to 7.4;
(i) Starting harvesting and liquid change operations when a liquid change trigger condition is met,

Here, the liquid change trigger condition includes:
(s1) Real-time pH ≤6.9, preferably ≤7.0, more preferably ≤7.05;
(s2) By adjusting the concentrations of oxygen, air, nitrogen and/or CO₂, the real-time pH value still decreases; and
(s3) Subculture time ≥72 h;

Here, the liquid change operation includes: recovering the cell-free virus-containing feed liquid in the culture, wherein the volume of the culture before recovery of the feed liquid is Vq2, the volume of the culture after recovery of the supernatant is Vh2 and the Vq2/Vh2 ratio is 3 to 15 (preferably 4 to 7, more preferably 5 to 6);
(j) Repeating the steps (h) and (i) m times, where m is 1, 2 or 3, wherein before the repetition, the culture solution is replenished to the culture container to form a transfected cell culture;
(k) Combining each of the recovered liquids to obtain combined virus-containing supernatant; and
(1) Purifying the combined virus-containing supernatant to obtain purified lentiviral vectors;

Here, the culture solution used in each of the above steps is a serum-free cell culture solution.

In another preferred embodiment, in the step (d), the volume of the packaging cell culture is Vb1 and the Vb1: Vq1 ratio is (0.8-1.2): 1.

In another preferred embodiment, in the step (j), the volume of the transfected cell culture is Vb2 and the Vb2: Vq2 ratio is (0.8-1.2): 1.

In another preferred embodiment, in the steps (c) and/or (h), culturing is conducted under a shaking condition.

In another preferred embodiment, the shaking condition is rocking back and forth 10 to 30 times per minute.

In another preferred embodiment, the amplitude of each rocking is 1 to 20 cm, preferably 5 to 10 cm.

In another preferred embodiment, the culture solutions added in different steps are same or different culture solutions.

In another preferred embodiment, in the step (c) and/or (h), the pH value is maintained in a range of 7.0 to 7.35.

In another preferred embodiment, in the steps (c) and/or (h), the dissolved oxygen is maintained in a range of 30% to 50%.

In another preferred embodiment, in the steps (c) and/or (h), the CO₂ concentration is maintained in a range of 3% to 5%.

In another preferred embodiment, in the steps (d) and/or (i), the Vql/Vhl ratio is 5 to 10.

In another preferred embodiment, the total time of the steps (c), (d) and (e) is 72 to 216 h. (Before transfection)

In another preferred embodiment, the total time of the steps (f), (g), (h), (i) and (j) is 72 to 120 h. (After transfection)

In another preferred embodiment, in the step (f), the multi-plasmid transfection used includes three-plasmid transfection and four-plasmid transfection.

In another preferred embodiment, the four-plasmid transfection is transfection using plasmid CAR, gag/pol, rev and VSVG.

In another preferred embodiment, the culture container is a disposable culture container.

In another preferred embodiment, the volume of the culture container is 20 to 120 L, preferably 30 to 100 L, more preferably 50 to 80 L.

In another preferred embodiment, the total number of viruses contained in the combined virus-containing supernatant is 1 × 10¹² Tu.

In another preferred embodiment, the virus titer in the combined virus-containing supernatant is 8 × 10⁷ Tu/ml.

In another preferred embodiment, the packaging cells are human embryonic renal epithelial cells.

In another preferred embodiment, the packaging cells are human embryonic renal epithelial cells HEK293F or cells derived therefrom.

In another preferred embodiment, the serum-free medium is LV-MAX™ Production Medium (Gibco™).

In another preferred embodiment, in the step (1), the purification includes: ultrafiltration and chromatography.

It should be understood that within the scope of the present invention, the above technical features of the present invention and the technical features described in detail below (e.g., embodiments) can be combined with each other to form new or preferred technical solutions. Due to space limitations, they are not described here.

### Detailed Description

After extensive and in-depth research, through exploration to production process and screening of process parameters, the inventors developed a method for producing lentivirus from GMP-level serum-free suspension cells on a large scale. The method provided by the present invention not only is suitable for large-scale production of 50 liters or more but also prepares high titer lentivirus in an extremely efficient manner. Further, because the whole process adopts serum-free culture conditions, the method avoids the risk of introducing animal-based proteins and other contaminants due to the use of serum. Further, the fluctuations among batches of lentiviruses produced by the method provided by the present invention are extremely small, which can meet the high requirements of GMP production for production quality. On this basis, the present invention is completed.

### Terminology

As used herein, terms "packaging cells of the present invention," "packaging cell HEK293F," and "packaging cell HEK293F of the present invention" can be used in an interchangeable manner and refer to the cells described in the first aspect of the present invention and used for packaging and producing lentiviruses.

### Packaging cells and packaging system

In the present invention, there is no particular limitation to the lentivirus packaging systems that can be used. Preferably, three-plasmid and four-plasmid systems can be used.

A particularly preferred packaging system uses lentiviral vectors derived from HIV-1, wherein a four-plasmid system is used instead of a three-plasmid system, the tat regulatory gene is knocked out and the gag/pol and rev carrier plasmids are split from one into two, thereby reducing the possibility of generating replicating viruses and greatly increasing the safety of the vector system.

In a preferred embodiment of the present invention, by using a four-plasmid system for lentivirus production, the safety and reliability of lentivirus products are further ensured.

### The present invention has the following main advantages:

(a) The cells of the present invention are suspension culture cells, which can be scaled up with the help of an automatic control system, so that the recombinant lentiviral vector feed liquid can be produced on a large scale, putting the production cost under control.
(b) The cell culture process described in the present invention adopts a one-time culture technology and uses a serum-free and protein-free medium, which eliminates the risk of contamination of heterologous protein and mad cow disease virus in the final product and greatly improves the safety of clinical application of the product, thereby being used in the production of cellular or genetic pharmaceuticals.

Below the present invention is further illustrated in conjunction with specific embodiments. It should be understood that these embodiments are intended to illustrate the present invention and not to limit the scope of the present invention. The experimental methods without specific conditions in the following embodiments are generally in accordance with conventional conditions, such as the conditions described in Sambrook et al., Molecular Cloning: Laboratory Manual (New York: Cold Spring Harbor Laboratory Press, 1989), or in accordance with the conditions recommended by the manufacturer. Unless otherwise stated, the percentages and parts are percentages by weight and parts by weight.

### General methods and materials

Medium: Serum-free and protein-free medium with definite chemical composition.

Culture condition: CO₂ 3%-5%; temperature 30∼37°C.
(1) Preparation of transfection reagent:
   a) 1 × HBS (pH7.4) : Dissolve 8.76 g of NaCl in 900 ml of ultrapure water, add 20 ml of 1M HEPES, adjust the pH value to 7.4, set the volume to 1 L, and filter (0.2 µ m filter membrane) and store it at 4 °C for later use.
   b) Weigh 125 mg of PEI powder, dissolve it in 50 ml of 1 × HBS (pH7.4), filter it with a 0.2 µm filter membrane, and store it at 4°C for later use.

### Embodiment 1

### Method for large-scale production of lentivirus

The present invention provides a method for large-scale production of lentivirus by using GMP-level serum-free suspension cells.

The method provided by the present invention comprises the following steps:
(a) Providing a seed solution of packaging cells, which are packaging cells that grow in suspension, for the production of lentivirus;
(b) Inoculating the seed solution to a first culture solution in a culture container to obtain a first culture, wherein the first culture solution is a serum-free cell culture solution and the inoculation density is 1 × 10⁶ to 5 × 10⁶ cells/ml and the volume of the first culture solution is a predetermined large volume (e.g., 3 to 100 L);
(c) Subjecting the first culture to subculture of packaging cells under the set conditions of temperature 30°C to 38°C, dissolved oxygen 35% to 55%, CO₂ concentration 2% to 10% and pH 6.9 to 7.4;
(d) Starting a liquid change operation when a liquid change trigger condition is met,

Here, the liquid change operation includes: discharging the cell-free supernatant in the culture from the culture container; then replenishing the culture solution to the culture container to form a culture of packaging cells;
(e) Repeating the steps (c) and (d) n times, where n is 1, 2 or 3;
(f) Starting a transfection operation when a transfection trigger condition is met, Here, the transfection operation includes: mixing the production plasmid and the transfection reagent for the production of the lentivirus and adding them to the culture container, thereby introducing the packaging cells and forming transfected packaging cells;
(g) Optionally performing liquid change after transfection;
(h) Cultivating the transfected packaging cells under the set conditions of temperature 30°C to 37°C, dissolved oxygen 35% to 55%, CO₂ concentration 2% to 10% and pH 6.9 to 7.4;
(i) Starting harvesting and liquid change operations when a liquid change trigger condition is met,
Here, the liquid change operation includes: recovering the cell-free virus-containing feed liquid in the culture;
(j) Repeating the steps (h) and (i) m times, where m is 1, 2 or 3, wherein before the repetition, the culture solution is replenished to the culture container to form a transfection cell culture;
(k) Combining each of the recovered liquids to obtain combined virus-containing supernatant; and
(1) Purifying the combined virus-containing supernatant to obtain purified lentiviral vectors;

Here, the culture solution used in each of the above steps is a serum-free cell culture solution.

In the present invention, the liquid change trigger condition is optimized, which helps to reduce the quality fluctuations of each production batch and helps to obtain medical-grade lentiviruses with a high titer and a high yield. Typically, the liquid change trigger condition includes:
(s1) Real-time pH ≤6.9, preferably ≤7.0, more preferably ≤7.05;
(s2) By adjusting the concentrations of oxygen, air, nitrogen and/or CO₂, the real-time pH value still shows a downward trend; and
(s3) Subculture time ≥72 h;

In the present invention, the transfection trigger condition is optimized, which helps to reduce the quality fluctuations of each production batch and helps to obtain medical-grade lentiviruses with a high titer and a high yield. Typically, the transfection trigger condition includes:
(t1) The total cell quantity is 0.05∼2 × 10¹¹ cells;
(t2) The cell density is 0.5∼5×10⁶ cells/ml;
(t3) The cell viability is ≥90%; and
(t4) The total culture time is ≥72 h;

Typically, in a specific embodiment of the present invention, the method comprises the following steps:
Preparation of a seed solution;
Inoculation (inoculated to 25 L of culture solution), with inoculation density of 1-5×10⁶ cells/ml;
Subculture of packaging cells (in the period, pH and dissolved oxygen are used to automatically adjust the ventilation ratio of dissolved CO₂, nitrogen and air, as well as the start mode of liquid change), controlling pH at 6.9 to 7.4, preferably 7.0 to 7.3, more preferably 7.1 to 7.2;
The first liquid change (when pH is ≤6.9, preferably ≤7.0, liquid change can be triggered), for example 25 L→72 to 10 L (preferably 3 to 7 L);
Replenish the serum-free culture solution and continue to culture for 24 to 96 h, preferably 36 to 72 h, more preferably 40 to 60 h; after culturing for 300 h, the number of the cells is 5×10¹⁰;
Add plasmids to transfect the packaging cells with multiple plasmids;
Optional step: After transfection, culture for 2 to 10 h, preferably 4 to 6 h, and perform liquid change again (first discharge a certain amount of culture mixture and then add a serum-free culture solution);
Universal step: After transfection, continue to culture. When pH is ≤6.9, preferably ≤7.0, the first liquid change can be triggered after transfection, and during liquid change after transfection, the discharged liquid culture mixture (cell-free virus-containing supernatant) is recovered, recorded as a recovered liquid R1 and stored at 2°C to 8°C;
After liquid change, continue to culture. When the liquid change trigger condition is met, an i-th liquid change after transfection is performed, where i is 2, 3, 4 or 5; during liquid change each time, the cell-free virus-containing supernatant of the discharged liquid culture mixture is recovered and recorded as recovered liquid Ri. This step is repeated 1, 2 or 3 times;
Combine each of the recovered liquids to obtain the combined virus-containing supernatant;
Purify the combined virus-containing supernatant to obtain purified lentiviral vectors, with a titer of 1×10⁸ to 1×10⁹ Tu/mL.

### Comparative example 1

Repeat embodiment 1, except for the following differences: the initial pH value is 6.9 to 7.4, but in the subculturing process, the real-time pH value of the first culture is not monitored in real time, and instead, subculturing is conducted in 24 to 96 h.

Result: after culturing for 300 h, the number of the cells is 4×10⁹ (less than the number of the cells in the preferred embodiment).

### Comparative example 2

Repeat embodiment 1, except for the following differences: CO₂ content is not adjusted.

Result: The biological titer of lentivirus is 20% of that in the preferred embodiment.

All the documents mentioned in the present invention are cited as references in the present application, as if each document is individually cited as a reference. Further, it should be understood that after reading the above teaching content of the present invention, those skilled in the art can make various changes or modifications to the present invention and these equivalent forms also fall within the scope defined by the appended claims of the present application.

## Claims

1. A method for producing lentivirus from serum-free suspension cells, wherein the method comprises the following steps:
(a) providing a seed solution of packaging cells, which are packaging cells that grow in suspension, for the production of lentivirus;
(b) inoculating the seed solution to a first culture solution in a culture container to obtain a first culture, wherein the first culture solution is a serum-free cell culture solution and the inoculation density is 1×10⁶ to 5×10⁶ cells/ml and the volume of the first culture solution is 3 to 100 L;
(c) subjecting the first culture to subculture of packaging cells under the set conditions of temperature 30°C to 38°C, dissolved oxygen 35% to 55%, CO₂ concentration 2% to 10% and pH 6.9 to 7.4, monitoring the real-time pH value of the first culture in the subculture process and controlling the concentrations of dissolved oxygen and/or CO₂ based on the real-time pH value, thereby maintaining the pH value in a range of 6.9 to 7.4;
(d) starting a liquid change operation when a liquid change trigger condition is met,
here, the liquid change trigger condition including:
(s1) real-time pH ≤6.9, preferably ≤7.0, more preferably ≤7.05;
(s2) by adjusting the concentrations of oxygen, air, nitrogen and/or CO₂, the real-time pH value still shows a downward trend; and
(s3) subculture time ≥72 h;
here, the liquid change operation including: discharging the cell-free supernatant in the culture from the culture container, wherein the volume of the culture before discharge of the supernatant is Vq1, the volume of the culture after discharge of the supernatant is Vh1 and the Vql/Vhl ratio is 3 to 15; and then replenishing the culture solution to the culture container to form a culture of packaging cells;
(e) repeating the steps (c) and (d) n times, where n is 1, 2 or 3;
(f) starting a transfection operation when a transfection trigger condition is met here, the transfection trigger condition including:
(t1) the total cell quantity is 0.05∼2 × 10¹¹ cells;
(t2) the cell density is 0.5∼5×10⁶ cells/ml;
(t3) the cell viability is ≥90%; and
(t4) the total culture time is ≥72 h;
here, the transfection operation including: mixing the production plasmid and the transfection reagent for the production of the lentivirus and adding them to the culture container, thereby introducing the packaging cells and forming transfected packaging cells;
(g) optionally performing liquid change after transfection;
(h) cultivating the transfected packaging cells under the set conditions of temperature 30°C to 37°C, dissolved oxygen 35% to 55%, CO₂ concentration 2% to 10% and pH 6.9 to 7.4, monitoring the real-time pH value of the first culture in the subculture process and controlling the concentrations of dissolved oxygen and/or CO₂ based on the real-time pH value, thereby maintaining the pH value in a range of 6.9 to 7.4;
(i) starting harvesting and liquid change operations when a liquid change trigger condition is met,
here, the liquid change trigger condition including:
(s1) real-time pH ≤6.9;
(s2) By adjusting the concentrations of oxygen, air, nitrogen and/or CO₂, the real-time pH value still decreases; and
(s3) subculture time ≥72 h;
here, the liquid change operation including: recovering the cell-free virus-containing feed liquid in the culture, wherein the volume of the culture before recovery of the feed liquid is Vq2, the volume of the culture after recovery of the supernatant is Vh2 and the Vq2/Vh2 ratio is 3 to 15;
(j) repeating the steps (h) and (i) m times, where m is 1, 2 or 3, wherein before the repetition, the culture solution is replenished to the culture container to form a transfection cell culture;
(k) combining each of the recovered liquids to obtain combined virus-containing supernatant; and
(l) purifying the combined virus-containing supernatant to obtain purified lentiviral vectors;
here, the culture solution used in each of the above steps is a serum-free cell culture solution.

2. The method according to claim 1, wherein in the steps (c) and/or (h), culturing is conducted under a shaking condition.

3. The method according to claim 1, wherein in the step (c) and/or (h), the pH value is maintained in the range of 7.0 to 7.35.

4. The method according to claim 1, wherein in the steps (d) and/or (i), the Vql/Vhl ratio is 5 to 10.

5. The method according to claim 1, wherein the total time of the steps (c), (d) and (e) is 72 to 216 h.

6. The method according to claim 1, wherein in the step (f), the multi-plasmid transfection used includes three-plasmid transfection and four-plasmid transfection.

7. The method according to claim 1, wherein the culture container is a disposable culture container.

8. The method according to claim 1, wherein the total number of viruses contained in the combined virus-containing supernatant is 1×10¹² Tu.

9. The method according to claim 1, wherein the packaging cells are human embryonic renal epithelial cells.

10. The method according to claim 1, wherein in the step (1), the purification includes: ultrafiltration and chromatography.
